# EUROPÄISCHE PATENTSCHRIFT

(11) **EP 2 845 549 B1**
(45) Veröffentlichungstag und Bekanntmachung des Hinweises auf die Patenterteilung: **31.08.2016**
(21) Anmeldenummer: 13184190.0
(22) Anmeldetag: 12.09.2013
(51) Int. Cl.: A61B 17/295, A61B 18/14, A61B 17/29

(54) **Chirurgisches Instrument mit verbessertem Betätigungsgetriebe**
Surgical instrument with improved actuating mechanism
Instrument chirurgical doté d'un mécanisme d'actionnement amélioré

(30) Priorität: 10.09.2013 EP 13183639
(43) Veröffentlichungstag der Anmeldung: 11.03.2015
(73) Patentinhaber: ERBE Elektromedizin GmbH, 72072 Tübingen (DE)
(72) Erfinder: Schiele, Elias, 72072 Tübingen (DE)
(74) Vertreter: Rüger, Barthelt & Abel

(56) Entgegenhaltungen:
- EP-A1- 2 371 316
- WO-A1-02/080783
- DE-C1- 4 421 822
- US-A1- 2011 087 218
- US-A1- 2011 276 049
- US-A1- 2011 290 854
- US-A1- 2012 184 990

## Beschreibung

Die Erfindung betrifft ein Instrument, insbesondere ein elektrochirurgisches Instrument, mit einem Werkzeug, das Glieder aufweist, die nacheinander zu betätigen sind.

Im Folgenden beschreibt der Begriff "distal" stets den vom Anwender entfernten Teil des Instruments oder Bauteils, der Begriff "proximal" beschreibt stets den zum Anwender hin gerichteten näher liegenden Teil des Instruments oder Bauteils.

Solche elektrochirurgischen Instrumente sind beispielsweise aus der EP 2 371 316 A1 bekannt. Das Werkzeug ist dort ein Maul mit zwei Branchen, von denen wenigstens eine schwenkbeweglich ist. Die Branche wird durch ein Zugelement in Schließrichtung bewegt. Zwischen den Branchen ist ein Messer verschiebbar gelagert. Sind die Branchen geschlossen, wird das Messer aktiviert, indem es in distaler Richtung verschoben wird. Zur Betätigung des Messers dient ein Schiebeelement.

Das Zugelement und das Schiebeelement sind mit einem Betätigungsgetriebe verbunden, das in einem Gehäuse angeordnet ist. Als Antrieb ist ein Bedienhebel vorgesehen, der auf dem ersten Teil seines Bewegungswegs die Zugbewegung zum Schließen der Branche und auf dem zweiten Teil seines Bewegungswegs die Schiebebewegung zum Vorschieben des Messers erzeugt. Die Schließbewegung in der Branche wird dabei über ein Kniehebelgetriebe umgesetzt.

Aus der US 2011/0290854 A ist ein chirurgisches Instrument mit einem Werkzeug bekannt, das eine bewegliche Branche und ein linear bewegliches Messer aufweist. Der Schaft des Instruments trägt an seinem distalen Ende das Werkzeug, wobei sich durch den Schaft mindestens ein Zugelement zur Betätigung der Branche und ein Schiebeelement zur Betätigung des Messers erstrecken. Das proximale Ende des Schafts ist mit einem Gehäuse verbunden, an dem ein Bedienhebel zur Betätigung sowohl des Zugelements als auch des Schiebeelements vorgesehen ist. Zur Umsetzung der Bewegung des Bedienhebels in eine Zugbewegung des Zugelements ist ein Kulissengetriebe vorgesehen.

Aus der DE 44 21 822 C1 ist ein chirurgisches Instrument bekannt, das zum Schließen eines Werkzeugs und zur nachfolgenden Betätigung eines Messers ebenfalls ein Getriebe aufweist. Um die Messerbewegung während des Schließens des Werkzeugs zu verhindern, ist ein Rastmechanismus vorgesehen. In dem Hebelgetriebe ist eine Scheibe mit Langloch vorgesehen, wobei das Langloch einen Mitnehmer erst dann mitnimmt, wenn das Werkzeug geschlossen ist.

Es besteht der Wunsch nach verbesserter Kontrolle der Schließbewegung und der auf das Gewebe einwirkenden Kraft.

Diese Aufgabe wird mit dem chirurgischen Instrument nach Anspruch 1 erzielt.

Das erfindungsgemäße chirurgische Instrument wird durch Anspruch 1 definiert. Bevorzugte Ausführungsformen sind in den abhängigen Ansprüchen gegeben. Die zweiteilige Form des Ansruchs 1 basiert auf US2011/0290854 A1. Es weist ein Kulissengetriebe zur aufeinanderfolgenden Umsetzung der Bewegung des Bedienhebels in eine Bewegung des Zugelements und in eine entgegengesetzte Bewegung des Schiebeelements auf. Die Erzeugung der Bewegung erfolgt mittels einer Kulisse und eröffnet damit einen weiten Gestaltungsspielraum hinsichtlich des Verhältnisses der Schwenkbewegung des Bedienhebels zur Schließbewegung der Branchen eines Werkzeugs. Das Bewegungsgesetz ist nicht durch eine Kniehebelanordnung vorgegeben, sondern kann durch die Wahl der Form der Kulisse weitgehend frei gewählt werden. In Verbindung mit einer Federanordnung zwischen dem Getriebe und dem einen oder mehreren Zugelementen zur Betätigung einer oder beiden Branchen kann die zwischen beiden Branchen wirkende Schließkraft genau festgelegt und dabei sichergestellt werden, dass sie unter verschiedenen Betriebsbedingungen auch mit unterschiedlichen Gewebeeigenschaften eingehalten wird.

Der Betätigungshebel weist außerdem eine Sperrkulisse auf, die eine Bewegung des Messers so lange sperrt, wie die Branchen nicht vollständig geschlossen sind. Dies ergibt insgesamt einen einfachen und übersichtlichen Aufbau und eine sichere Funktion unter Zulassung der in der Kunststofftechnik üblichen Toleranzen.

Einzelheiten vorteilhafter Ausführungsformen der Erfindung ergeben sich aus Unteransprüchen, der Beschreibung und/oder der Zeichnung. Es zeigen:
Figur 1 das erfindungsgemäße Instrument in einer Gesamtansicht in Prinzipdarstellung,
Figur 2 das Werkzeug des Instruments nach Figur 1, in schematisierter Prinzipdarstellung,
Figur 3 das Getriebe des Instruments nach Figur 1, in schematisierter Prinzipdarstellung,
Figur 4 das Instrument nach Figur 1 mit geöffnetem Gehäuse, in Ausgangsposition,
Figur 5 das Instrument nach Figur 4 in der Betätigungsposition für das geschlossene Werkzeug vor Betätigung des Messers und
Figur 6 das Instrument nach Figur 4 und 5 bei betätigtem Messer.

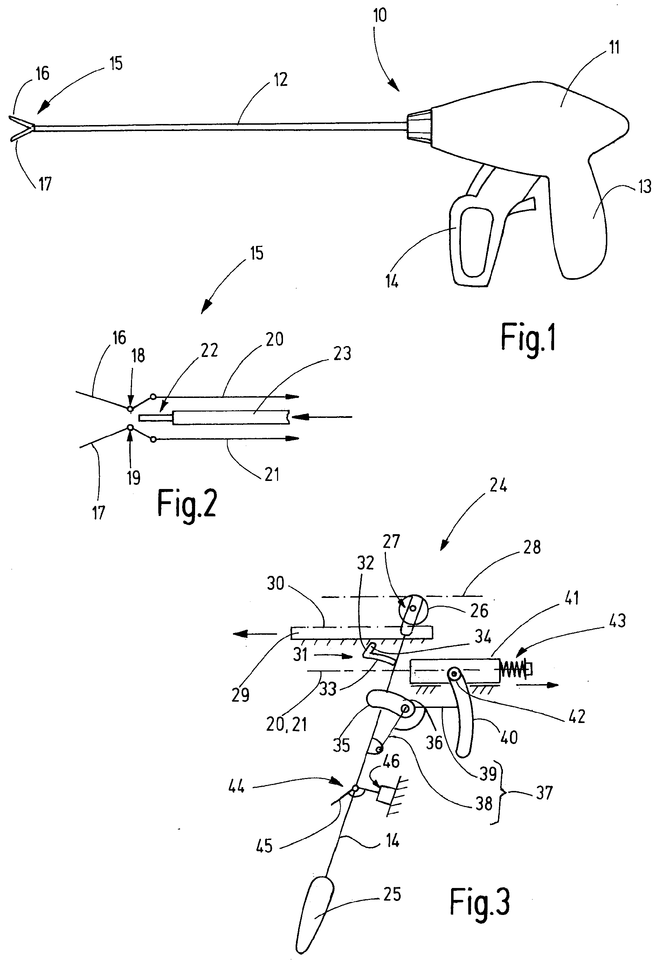

In Figur 1 ist ein Instrument 10 schematisch veranschaulicht, das ein Gehäuse 11 mit einem sich von diesem wegerstreckenden Schaft 12 aufweist. An dem Gehäuse 11 ist ein Handgriff 13 ausgebildet, in dessen Nachbarschaft ein Bedienhebel 14 angeordnet ist.

An dem distalen Ende des Schafts 12 ist ein Werkzeug 15 angeordnet, das zwei Branchen 16, 17 aufweist. Von diesem ist mindestens eine schwenkbeweglich gelagert, so dass das zwischen den Branchen 16, 17 gebildete Maul geöffnet und geschlossen werden kann.

Figur 2 veranschaulicht diesen Vorgang schematisch. Die Branchen 16, 17 des Werkzeugs 15 sind durch geeignete, nicht weiter veranschaulichte Schwenklager 18, 19 aufeinander zu und voneinander weg schwenkbeweglich gelagert. Zur Betätigung setzen an den proximalen Enden der Branchen 16, 17 Zugelemente 20, 21 an, die sich durch den Schaft 12 hindurch bis in das Gehäuse 11 hinein erstrecken.

Zu dem Werkzeug 15 gehört außerdem ein Messer 22 das schiebebeweglich gelagert ist, um in eine von den Branchen 16, 17 festgelegte gerade oder gekrümmte Messerführungsnut einzufahren und zwischen den Branchen 16, 17 gefasstes Gewebe zu durchtrennen. Das Messer 22 schneidet dabei zum Beispiel mit seiner quer zur Bewegungsrichtung orientierten Stirnkante. Es wird durch ein Schiebeelement 23 bewegt, das sich vorzugsweise zwischen den Zugelementen 20, 21 durch den Schaft 12 bis in das Gehäuse 11 hinein erstreckt.

Das in dem Gehäuse 11 angeordnete Getriebe 24 zur Umsetzung der Bewegung des Bedienhebels 14 in eine Zugbewegung der Zugelemente 20, 21 und eine Schubbewegung des Schiebeelements 23 ist in Figur 3 gesondert schematisch veranschaulicht. Der Bedienhebel 14 weist unten eine Greiföffnung 25 auf. An seinem anderen Ende steht er mit einem Zahnrad 26 in Eingriff, das an dem Bedienhebel 14 drehbar und in Hebellängsrichtung verschiebbar gelagert ist. Der Bedienhebel 14 weist zur Aufnahme der Achse des Zahnrads 26 beispielsweise eine Führung für das Zahnrad beispielsweise in Form eines Langlochs oder eines gegabelten Endes 27 auf.

Das Zahnrad 26 steht mit einer an dem Gehäuse 11 ortsfest ausgebildeten Zahnstange 28 in Eingriff. An seiner gegenüberliegenden Seite steht das Zahnrad 26 mit einer Zahnstange 30 eines Schlittens 29 in Verbindung. Diese Zahnstange bildet eine Schlittenverzahnung 30, die sich parallel zu der Zahnstange 28 erstreckt. Der Schlitten 29 ist parallel zu der Zahnstange 28 bewegbar und dient zur Betätigung des Messers 22. Die Zahnstange 28, das Zahnrad 26 und die Zahnstange 30 des Schlittens 29 bilden ein Zahnstangengetriebe.

An dem Bedienhebel 14 ist außerdem eine Sperrkulisse 31 ausgebildet, die zwei im Winkel zueinander stehende Abschnitte 32, 33 aufweist. Die Sperrkulisse 31 ist von einem Stift 34 durchgriffen, der in dem Gehäuse 11 ortsfest angeordnet ist. Die Sperrkulisse 31 bewegt sich mit dem Bedienhebel 14 so, dass sich bei der Betätigung der Branchen 16, 17 der Stift 34 in dem ersten Abschnitt 32 befindet. Bei der Messerbetätigung befindet sich der Stift 34 in dem zweiten Abschnitt 33.

Ein erster Arm 38 des Hebels 37 ist gelenkig mit dem Bedienhebel 14 verbunden. Sein zweiter Arm 39 trägt wiederum eine Kulisse 40, die zur Betätigung eines Schlittens 41 für die Zugelemente 20, 21 dient. Die gebogene, sich quasi quer zu dem zweiten Arm 39 erstreckende Kulisse 40 führt einen Kulissenzapfen 42, der mit dem Schlitten 41 verbunden ist. Der Schlitten 41 ist über ein Federelement 43, wie beispielsweise eine Druckfeder, mit den durch eine gestrichelte Linie angedeuteten Zugelementen 20, 21 verbunden.

Zur Vermeidung einer unerwünschten Betätigung des Messers 22 ist eine Sperreinrichtung 44 vorgesehen. Zu dieser gehören ein oder mehrere Sperrhebel 45, denen ein an dem Gehäuse 11 ausgebildeter Anschlag 46 zugeordnet ist. Der Sperrhebel 45 kann durch Handbetätigung mit dem Anschlag 46 außer Eingriff gebracht werden, um die Messerbewegung freizugeben. Vorzugsweise läuft der Sperrhebel 45 bei einer Betätigung des Bedienhebels 14 dann gegen den Anschlag 46, wenn der Stift 34 zwischen den beiden Abschnitten 32, 33 der Sperrkulisse 31 steht.

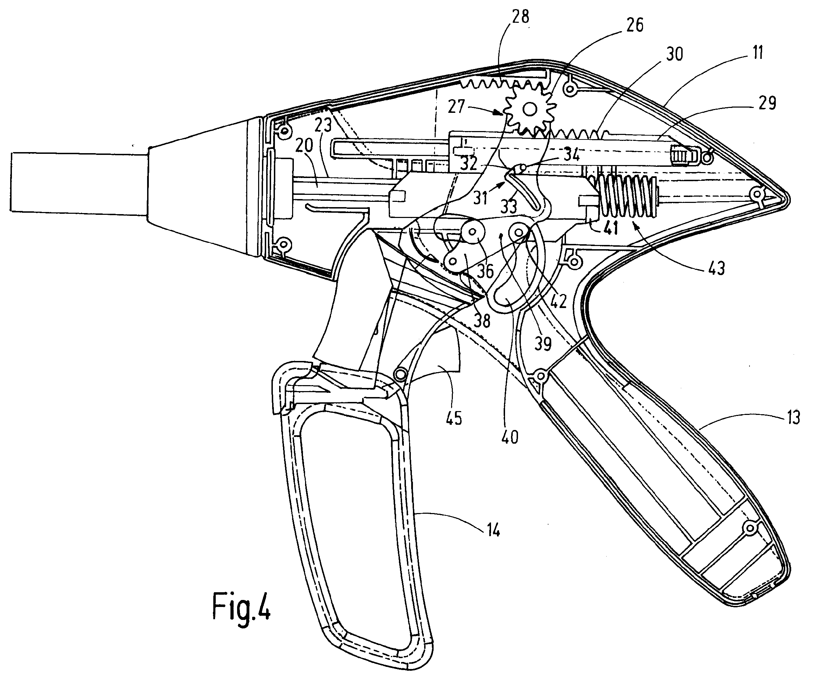

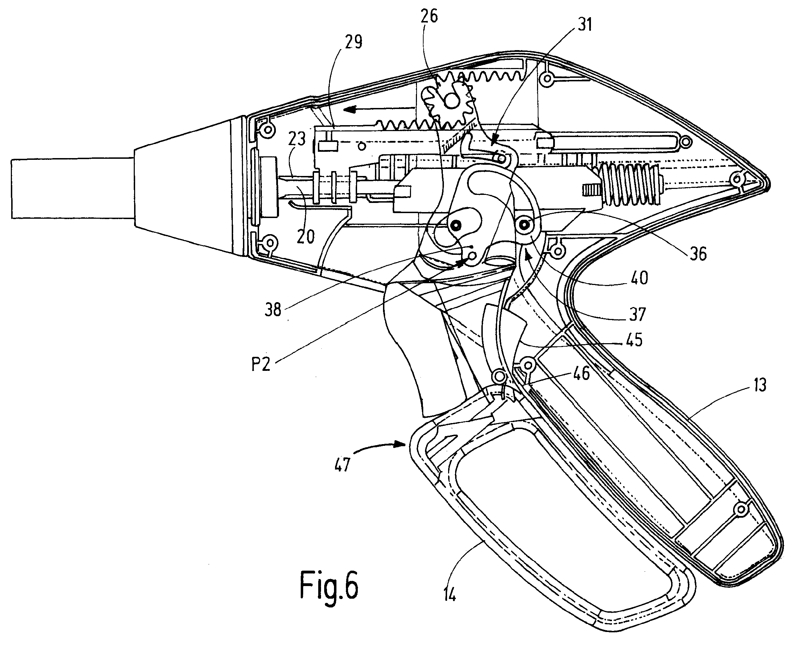

Das Instrument 10 arbeitet unter nachfolgender Bezugnahme auf die Figuren 4 bis 6 wie folgt:
Im Ausgangszustand ist der Bedienhebel 14 in distaler, das heißt in Figur 4, linker Position. Er steht dabei so, dass die Sperrkulisse 31 den Stift 34 mit dem oberen Ende ihres Abschnitts 32 aufnimmt. Beide Schlitten 29, 41 befinden sich in Ruheposition.

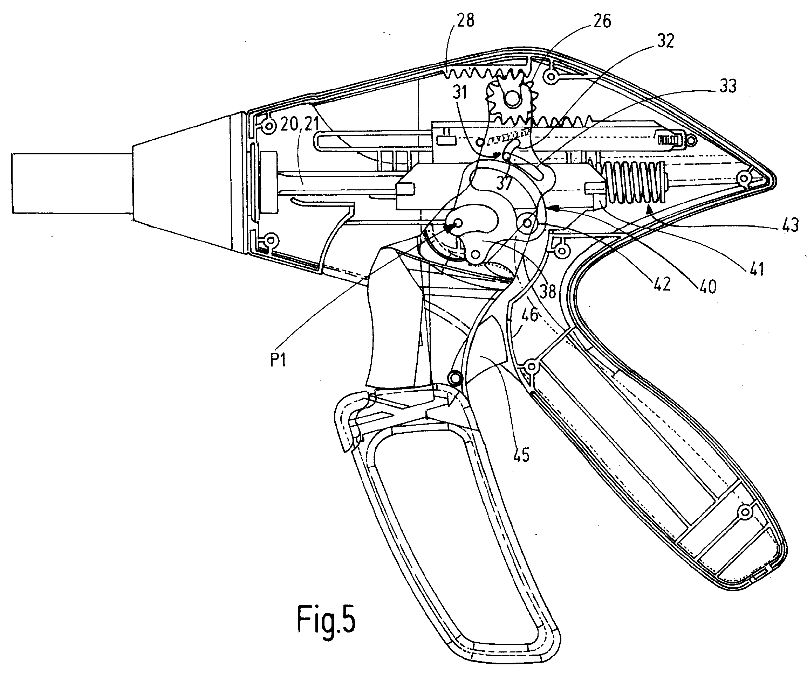

Wird der Bedienhebel 14 nun auf den Handgriff 13 hin bewegt, wird der Bedienhebel 14 durch den Abschnitt 32 der Sperrkulisse 31 daran gehindert, mit seinem oberen Ende merklich in distaler Richtung zu schwenken. Er wird deswegen am oberen Ende in der in den Figuren 4 und 5 rechten Position, das heißt oben in seiner Proximalposition festgehalten, während er eine erste Schwenkbewegung vollführt. Dabei ist er über den Arm 38 an das Zentrum des Kulissenzapfens 36 als (erster, vorläufiger) Schwenk- oder Drehpunkt P1 angebunden.

Durch die Schwenkbewegung des Bedienhebels 14 um den Schwenk- oder Drehpunkt P1 wird der Bedienhebel 14 etwas in Vertikalrichtung (d.h. in seiner Längsrichtung) verstellt. Dadurch gelangt die Sperrkulisse 31 nach oben, bis der Stift 34 in den Winkel der Sperrkulisse 31, das heißt zwischen den Abschnitten 32, 33 steht. Der Sperrhebel 45 läuft dabei an dem Anschlag 46 an und blockiert zunächst jede weitere Bewegung.

Die Schwenkbewegung des Bedienhebels 14 hat bei der oben beschriebenen ersten Etappe der Schwenkbewegung dazu geführt, dass der zweiarmige Hebel 37 geschwenkt und damit die Kulisse 40 entlang ihrer gesamten Länge an dem Kulissenzapfen 42 entlanggelaufen ist. Weil die Anordnung der Führungsbahn der Kulisse 40 entlang ihrer Länge unterschiedliche Abstände zu dem Drehpunkt P1 aufweist, ist dadurch der Schlitten 41 in proximaler Richtung bewegt worden, so dass die Zugelemente 20, 21 eine Zugbewegung auf das Werkzeug 15 übertragen haben. Das Bewegungsgesetz der Schlittenbewegung ergibt sich aus der geeignet festgelegten Form der Kulisse 40.

Der gesamte Kulissenweg beinhaltet zwei Funktionen. Im ersten Abschnitt wird der Schlitten 41 bewegt und die Branchen 16, 17 geschlossen. Die Längsbewegung des Schlittens 41 ist abhängig von dem Volumen des zwischen den Branchen 16, 17 gefassten Gewebes. Im zweiten Abschnitt des Kulissenwegs werden die Branchen 16, 17 geklemmt. Abhängig von dem Volumen des zwischen den Branchen 16, 17 gefassten Gewebes ändern sich die Längen der beiden Abschnitte (Verfahr- bzw. Klemmabschnitt). Wird beispielsweise zwischen den Branchen 16, 17 kein Gewebe gefasst, umfasst der Abschnitt, in dem die Branchen 16, 17 geklemmt werden, lediglich den letzten Bereich des Kulissenwegs der Kulisse 40. Wird großvolumiges Gewebe zwischen den Branchen 16, 17 gefasst, kann der Abschnitt des Kulissenwegs, der die Klemmkraft aufbringt, nahezu den gesamten Kulissenweg umfassen. Der zweiarmige Hebel 37, die Kulisse 40 und der Kulissenzapfen 42 bilden ein Kulissengetriebe. Das Federelement 43 ist vorgespannt. Dadurch wird sichergestellt, dass, nachdem der komplette Kulissenweg durchlaufen ist, das zwischen den Branchen 16, 17 gefasste Gewebe mit einer vordefinierten Mindestklemmkraft gehalten ist.

Will der Anwender nun das Messer 22 betätigen, muss er die Sperreinrichtung 44 lösen. Dazu schwenkt er beispielsweise durch einen geeigneten Hebelübertragungsmechanismus 47 den Sperrhebel 45 von dem Anschlag 46 weg, so dass eine weitere Bewegung des Bedienhebels 14 auf den Handgriff 13 hin freigegeben ist.

Die Kulisse 40 steht im Endanschlag und verhindert so ein weiteres Verschwenken des zweiarmigen Hebels 37. Damit wird nun der Verbindungspunkt zwischen dem ersten Arm 38 des Hebels 37 und dem Bedienhebel 14 zum neuen Drehpunkt P2. Die Sperrkulisse 31 ist ebenfalls freigegeben. Deswegen fährt nun das Zahnrad 26 an der Zahnstange 28 abrollend in distaler Richtung. Aufgrund der doppelten Umfangsgeschwindigkeit des Zahnrads 26 im Verhältnis zu dessen linearer Vorschubgeschwindigkeit wird der Schlitten 29 mit doppelter Geschwindigkeit und dementsprechend doppeltem Hub in diese Richtung befördert. Das Schiebeelement 23 wird auf diese Weise sehr schnell vorgeschoben.

Bei dem vorgestellten Kulissengetriebe lassen sich die Bewegungsgesetze durch Anordnung und Ausbildung der beteiligten zwei Kulissen 31 und 40 in weiten Grenzen relativ frei gestalten. Durch den Wechsel des Drehpunkts von P1 nach P2 lassen sich außerdem zusätzlich zu den sonst vorhandenen Unterschieden zusätzliche Übersetzungsunterschiede zwischen der Betätigung der Branchen 16, 17 und des Messers 22 erzeugen. Es kann so eine örtlich und kräftemäßig gut kontrollierte Bewegung der Branchen 16, 17 und eine schnelle Schneidebewegung des Messers 22 erzeugt werden.

Ein erfindungsgemäßes Instrument 10 weist ein Kulissengetriebe mit einer Sperrkulisse 31 und eine zur Kraftübertragung und Steuerung dienenden Kulisse 40 auf, um nacheinander zunächst eine Zugbewegung an Zugelementen 20, 21 und dann eine Schiebebewegung an einem Schiebeelement 23 zu erzeugen. Das Konzept gestattet einen toleranzunempfindlichen funktionssicheren Aufbau.

### Bezugszeichenliste:

- 10: Instrument
- 11: Gehäuse
- 12: Schaft
- 13: Handgriff
- 14: Bedienhebel
- 15: Werkzeug
- 16, 17: Branchen
- 18, 19: Schwenklager
- 20, 21: Zugelemente
- 22: Messer
- 23: Schiebeelement
- 24: Getriebe
- 25: Greiföffnung
- 26: Zahnrad
- 27: gegabeltes Ende
- 28: Zahnstange am Gehäuse 11
- 29: Schlitten
- 30: Schlittenverzahnung
- 31: Sperrkulisse
- 32, 33: Abschnitte der Sperrkulisse 31
- 34: Stift
- 36: Kulissenzapfen
- 37: zweiarmiger Hebel
- 38: erster Arm des Hebels 37
- 39: zweiter Arm des Hebel 37
- 40: Kulisse
- 41: Schlitten
- 42: Kulissenzapfen
- 43: Federelement
- 44: Sperreinrichtung
- 45: Sperrhebel
- 46: Anschlag
- 47: Hebelübertragungsmechanismus
- P1: erster temporärer Drehpunkt für Branchenbewegung
- P2: zweiter temporärer Drehpunkt für Messerbewegung

## Patentansprüche

1. Chirurgisches Instrument (10),
mit einem Werkzeug (15), das mindestens eine bewegliche Branche (16, 17) und mindestens ein linear bewegliches Messer (22) aufweist,
mit einem Schaft (12), der an seinem distalen Ende das Werkzeug (15) trägt und durch den sich mindestens ein Zugelement (20, 21) zur Betätigung der Branche (16, 17) und ein Schiebeelement (23) zur Betätigung des Messers (22) erstrecken,
mit einem Gehäuse (11), das mit einem anderen, proximalen Ende des Schafts (12) verbunden ist,
mit einem Bedienhebel (14) zur Betätigung sowohl des Zugelements (20, 21) als auch des Schiebeelements (23),
mit einem Kulissengetriebe (37, 40, 42) zur Umsetzung der Bewegung des Bedienhebels (14) in eine Zugbewegung des Zugelements (20, 21),
**dadurch gekennzeichnet dass** der Betätigungshebel eine Sperrkulisse (31) zum Sperren einer Bewegung des Schiebeelements (23) bei Schwenkung des Bedienhebels (14) um einen ersten Hebeldrehpunkt (P1) während des Schließens des Werkzeugs (15) sowie zur Freigabe einer Bewegung des Schiebeelements (23) bei weiterer Schwenkung des Bedienhebels (14) nach Schließen des Werkzeugs (15) zur Betätigung des Messers (22) aufweist.

2. Instrument nach Anspruch 1, **dadurch gekennzeichnet, dass** ein Zahnstangengetriebe (28, 26, 30) vorgesehen ist, das zur Umsetzung einer Schwenkbewegung des Bedienhebels (14) in eine Schiebebewegung des Schiebeelements (23) eingerichtet ist.

3. Instrument nach einem der vorigen Ansprüche, **dadurch gekennzeichnet, dass** die Sperrkulisse (31) durch einen in dem Bedienhebel (14) ausgebildeten Schlitz mit zwei in einem Winkel zueinander stehenden Abschnitten (32, 33) gebildet ist, wobei der Schlitz von einem bezüglich des Gehäuses (11) ortsfesten Stift (34) durchgriffen ist.

4. Instrument nach einem der vorigen Ansprüche, **dadurch gekennzeichnet, dass** zu dem Kulissengetriebe (37, 40, 42) ein zweiarmiger Hebel (37) gehört, der um ein bezüglich des Gehäuses (11) ortsfestes Lager schwenkbar ist, das einen Drehpunkt (P1) festlegt.

5. Instrument nach einem der vorigen Ansprüche, **dadurch gekennzeichnet, dass** der Bedienhebel (14) endseitig mit einem Zahnrad (26) in Verbindung steht, das mit einer bezüglich des Gehäuses (11) ortsfesten Zahnstange (28) in Eingriff steht.

6. Instrument nach Anspruch 5, **dadurch gekennzeichnet, dass** das Zahnrad (26) mit einer linearen Schlittenverzahnung (30) in Eingriff steht, die parallel zu der Zahnstange (28) verläuft und an einem parallel zu der Zahnstange (28) verschiebbar angeordneten Schlitten (29) ausgebildet ist.

7. Instrument nach Anspruch 4, **dadurch gekennzeichnet, dass** der zweiarmige Hebel (37) einen ersten Arm (38) aufweist, der mit dem Bedienhebel (14) gelenkig verbunden ist.

8. Instrument nach Anspruch 7, **dadurch gekennzeichnet, dass** der zweiarmige Hebel (37) einen zweiten Arm (39) aufweist, in dem eine Kulisse (40) ausgebildet ist.

9. Instrument nach Anspruch 8, **dadurch gekennzeichnet, dass** die Kulisse (40) mit einem den Branchen (16, 17) zugeordneten Schlitten (41) in Verbindung steht.

10. Instrument nach Anspruch 9, **dadurch gekennzeichnet, dass** durch die Anordnung der Kulisse (40) der Betätigungshub des den Branchen (16, 17) zugeordneten Schlittens (41) begrenzt ist.

11. Instrument nach Anspruch 9 oder 10, **dadurch gekennzeichnet, dass** der den Branchen (16, 17) zugeordnete Schlitten (41) über ein Federelement (43) mit den Zugelementen (20, 21) verbunden ist.

12. Instrument nach einem der vorigen Ansprüche, **dadurch gekennzeichnet, dass** dem Bedienhebel (14) eine Sperreinrichtung (44) zugeordnet ist, die einen Sperrpunkt festlegt, um die Betätigungsbewegung auf einen Teilweg zu begrenzen.

13. Instrument nach Anspruch 3 und 12, **dadurch gekennzeichnet, dass** der Sperrpunkt mit dem Punkt der Bewegung des Bedienhebels (14) übereinstimmend festgelegt ist, an dem der Stift (34) in der Sperrkulisse (31) zwischen beiden Abschnitten (32, 33) der Sperrkulisse 31 steht.

14. Instrument nach einem der vorigen Ansprüche, **dadurch gekennzeichnet, dass** das Kulissengetriebe (37, 40, 42) dem Bedienhebel (14) zwei unterschiedliche temporäre Drehpunkte (P1, P2) zuordnend ausgebildet ist, wobei ein Drehpunkt (P1) der Bewegung der Branchen (16, 17) und der andere Drehpunkt (P2) der Bewegung des Messers (22) zugeordnet ist.

## Claims

1. Surgical instrument (10),
having a tool (15) which has at least one moveable branch (16, 17) and at least one linearly moveable blade (22), having a shaft (12) which bears the tool (15) on its distal end and through which at least one tension element (20, 21) to actuate the branch (16, 17) and a sliding element (23) to actuate the blade (22) are extended, having a housing (11) which is connected to another, proximal end of the shaft (12),
having an operating lever (14) to actuate both the tension element (20, 21) and the sliding element (23),
having a gate-type gear (37, 40, 42) to convert the movement of the operating lever (14) into a tension movement of the tension element (20, 21),
**characterised in that** the actuating lever has a blocking guide (31) to block a movement of the sliding element (23) in the case of pivoting of the operating lever (14) about a first lever rotational point (P1) during the closing of the tool (15) as well as to release a movement of the sliding element (23) in the case of further pivoting of the operating lever (14) after closing of the tool (15) to actuate the blade (22).

2. Instrument according to claim 1, **characterised in that** a rack-and-pinion gear (28, 26, 30) is provided which is established to convert a pivot movement of the operating lever (14) into a sliding movement of the sliding element (23).

3. Instrument according to one of the preceding claims, **characterised in that** the blocking guide (31) is formed by a slot formed in the operating lever (14) having two sections (32, 33) which are at an angle to each other, wherein the slot is passed through by a pin (34) which is fixed in place with regard to the housing (11).

4. Instrument according to one of the preceding claims, **characterised in that** a two-armed lever (37) belongs to the gate-type gear (37, 40, 42), said lever being able to pivot about a bearing which is fixed in place with regard to the housing (11), said bearing determining a rotational point (P2).

5. Instrument according to one of the preceding claims, **characterised in that** the operating lever (14) is connected to a gearwheel (26) at an end side, said gearwheel engaging with a gear rack (28) which is fixed in place with regard to the housing (11).

6. Instrument according to claim 5, **characterised in that** the gearwheel (26) engages with a linear slide toothing (30) which runs in parallel to the gear rack (28) and is formed on a slide (29) which is arranged to be able to move in parallel to the gear rack (28).

7. Instrument according to claim 4, **characterised in that** the two-armed lever (37) has a first arm (38) which is connected supplely to the operating lever (14).

8. Instrument according to claim 7, **characterised in that** the two-armed lever (37) has a second arm (39) in which a sliding block (40) is formed.

9. Instrument according to claim 8, **characterised in that** the sliding block (40) is connected to a slide (41) allocated to the branches (16, 17).

10. Instrument according to claim 9, **characterised in that** the actuating stroke of the slide (41) allocated to the branches (16, 17) is limited by the arrangement of the sliding block (40).

11. Instrument according to claim 9 or 10, **characterised in that** the slide (41) allocated to the branches (16, 17) is connected to the tension elements (20, 21) via a spring element (43).

12. Instrument according to one of the preceding claims, **characterised in that** a blocking device (44) is allocated to the operating lever (14), said blocking device determining a blocking point in order to limit the actuation movement to a partial path.

13. Instrument according to claim 3 and 12, **characterised in that** the blocking point is determined to correspond to the point of the movement of the operating lever (14) at which the pin (34) is in the blocking guide (31) between both sections (32, 33) of the blocking guide (31).

14. Instrument according to one of the preceding claims, **characterised in that** the gate-type gear (37, 40, 42) is formed to allocate two different temporary rotational points (P1, P2) to the operating lever (14), wherein one rotational point (P1) is allocated to the movement of the branches (16, 17) and the other rotational point (P2) to the movement of the blade (22).

## Revendications

1. Instrument chirurgical (10),
comprenant un outil (15) qui présente au moins un mors (16, 17) mobile et au moins un couteau (22) déplaçable de façon linéaire,
comprenant une tige (12) qui porte l'outil (15) à son extrémité distale et dans laquelle s'étendent au moins un élément de traction (20, 21), destiné à l'actionnement du mors (16, 17), et un élément coulissant (23) destiné à actionner le couteau (22),
comprenant un boîtier (11) qui est relié à une autre extrémité, à savoir l'extrémité proximale, de la tige (12),
comprenant un levier de commande (14) destiné à l'actionnement aussi bien de l'élément de traction (20, 21) que de l'élément coulissant (23),
comprenant un mécanisme à coulisse (37, 40, 42) destiné à transformer le mouvement du levier de commande (14) en un mouvement de traction de l'élément de traction (20, 21),
**caractérisé en ce que** le levier d'actionnement présente une coulisse d'arrêt (31) pour le blocage d'un mouvement de l'élément coulissant (23), lors du pivotement du levier de commande (14) autour d'un premier point d'articulation de levier (P1), pendant la fermeture de l'outil (15), ainsi que pour la libération d'un mouvement de l'élément coulissant (23) lors du pivotement ultérieur du levier de commande (14), après la fermeture de l'outil (15), en vue de l'actionnement du couteau (22).

2. Instrument selon la revendication 1, **caractérisé en ce qu'**il est prévu un mécanisme à crémaillère (28, 26, 30) qui est agencé pour transformer un mouvement pivotant du levier de commande (14) en un mouvement coulissant de l'élément coulissant (23).

3. Instrument selon l'une des revendications précédentes, **caractérisé en ce que** la coulisse d'arrêt (31) est formée par une fente ménagée dans le levier de commande (14) et comportant deux parties (32, 33) formant un angle entre elles, la fente étant traversée par un doigt (34) qui est fixe par rapport au boîtier (11).

4. Instrument selon l'une des revendications précédentes, **caractérisé en ce que** le mécanisme à coulisse (37, 40, 42) comprend un levier (37) à deux bras pouvant pivoter autour d'un palier qui est fixe par rapport au boîtier (11) et définit un point d'articulation (P1).

5. Instrument selon l'une des revendications précédentes, **caractérisé en ce que** le levier de commande (14) est relié par son extrémité à un pignon (26) qui engrène avec une crémaillère (28) fixe par rapport au boîtier (11).

6. Instrument selon la revendication 5, **caractérisé en ce que** le pignon (26) engrène avec une denture de chariot (30) linéaire qui s'étend parallèlement à la crémaillère (28) et est réalisée sur un chariot (29) disposé de manière à pouvoir être déplacé parallèlement à la crémaillère (28).

7. Instrument selon la revendication 4, **caractérisé en ce que** le levier (37) à deux bras présente un premier bras (38) qui est articulé sur le levier de commande (14).

8. Instrument selon la revendication 7, **caractérisé en ce que** le levier (37) à deux bras présente un deuxième bras (39) dans lequel est réalisée une coulisse (40).

9. Instrument selon la revendication 8, **caractérisé en ce que** la coulisse (40) est reliée à un chariot (41) associé aux mors (16, 17).

10. Instrument selon la revendication 9, **caractérisé en ce que** du fait de la disposition de la coulisse (40), la course d'actionnement du chariot (41) associé aux mors (16, 17) est limitée.

11. Instrument selon la revendication 9 ou 10, **caractérisé en ce que** le chariot (41) associé aux mors (16, 17) est relié aux éléments de traction (20, 21) par l'intermédiaire d'un élément faisant ressort (43).

12. Instrument selon l'une des revendications précédentes, **caractérisé en ce qu'**il est associé au levier de commande (14), un dispositif d'arrêt (44) qui définit un point d'arrêt afin de limiter le mouvement d'actionnement à une course partielle.

13. Instrument selon les revendications 3 et 12, **caractérisé en ce que** le point d'arrêt est défini de manière à coïncider avec le point du mouvement du levier de commande (14), auquel le doigt (34) dans la coulisse d'arrêt (31) se trouve entre les deux parties (32, 33) de la coulisse d'arrêt (31).

14. Instrument selon l'une des revendications précédentes, **caractérisé en ce que** le mécanisme à coulisse (37, 40, 42) est réalisé de manière à associer deux points d'articulation (P1, P2) temporaires différents au levier de commande (14), sachant qu'un point d'articulation (P1) est associé au mouvement des mors (16, 17) et l'autre point d'articulation (P2) est associé au mouvement du couteau (22).
